# FASCICULE DE BREVET EUROPEEN

(11) **EP 0 672 406 B1**
(45) Date de publication et mention de la délivrance du brevet: **16.02.2000**
(21) Numéro de dépôt: 95400350.5
(22) Date de dépôt: 20.02.1995
(51) Int. Cl.: A61K 7/06, A61K 31/365

(54) **L'utilisation d'une composition à base d'alpha-pyrones pour induire et stimuler la croissance des cheveux et/ou freiner leur chute.**
Zusammensetzung auf der Basis von alpha-Pyronen zur Induzierung und Stimulierung des Haarwachstums und/oder zur Verlangsamung des Haarausfalls und ihre Verwendung
Use of a composition based on alpha-pyrones for inducing and stimulating hair growth and/or inhibit loss.

(30) Priorité: 15.03.1994 FR 9402992
(43) Date de publication de la demande: 20.09.1995
(73) Titulaire: L'OREAL, 75008 Paris (FR)
(72) Inventeur: Rosenbaum, Georges, F-92110 Clichy (FR)
(74) Mandataire: Tezier Herman, Béatrice

(56) Documents cités:
- WO-A-93/08800
- US-A- 4 139 619
- EUR. J. PHARMACOL., vol. 215, no. 2-3, 1992 pages 265-269, C. BACKHAUS ET AL. 'Extract of kava(Piper methysticum) and its methysticin constituents protect brain tissue against ischemic damage in rodents.'
- THERAPIEWOCHE, vol. 34, no. 27, 1984 pages 4117-4127, K. SCHIMMEL 'Pflanzliche sedativa.'

## Description

La présente invention concerne l'utilisation dans des compositions cosmétiques et/ou thérapeutiques à usage topique destinées à induire et stimuler la croissance des cheveux et/ou freiner leur chute, à titre de principe actif, d'au moins une α-Pyrone, ainsi que leur utilisation dans cette application.

Chez l'être humain, la croissance des cheveux et leur renouvellement sont principalement déterminés par l'activité des follicules pileux. Leur activité est cyclique et comporte essentiellement trois phases, à savoir la phase anagène, la phase catagène et la phase télogène.
A la phase anagène active ou phase de croissance, qui dure plusieurs années et au cours de laquelle les cheveux s'alllongent, succède une phase catagène très courte et transitoire, puis une phase de repos, appelée phase télogène, qui dure quelques mois.
A la fin de la période de repos, les cheveux tombent et un autre cycle recommence. La chevelure se renouvelle donc en permanence, et sur les 150 000 cheveux environ que comporte une chevelure, à chaque instant, 10% d'entre eux environ sont au repos et seront donc remplacés en quelques mois.
Dans un nombre important de cas, la chute précoce des cheveux survient chez des sujets prédisposés génétiquement et elle atteint notamment les hommes. Il s'agit plus particulièrement de l'alopécie androgénétique ou androgénique ou encore androgéno-génétique.
Cette alopécie est essentiellement due à une perturbation du renouvellement capillaire qui entraîne, dans un premier temps, l'accélération de la fréquence des cycles aux dépens de la qualité des cheveux puis de leur quantité. Il se produit un appauvrissement progressif de la chevelure par régression des cheveux dits "terminaux" au stade de duvets. Des zones sont touchées préférentiellement, notamment les golfes temporaux ou frontaux chez l'homme, et chez les femmes, on constate une alopécie diffuse du vertex.

On recherche depuis de nombreuses années, dans l'industrie cosmétique ou pharmaceutique, des compositions permettant de supprimer ou de réduire l'effet de l'alopécie andro-génétique, et notamment d'induire ou de stimuler la croissance des cheveux ou de diminuer leur chute.

Dans cette optique, on a certes déjà proposé un grand nombre de composés actifs très divers, comme par exemple le 6-amino 1,2-dihydro 1-hydroxy 2-imino 4-pipéridino pyrimidine et ses dérivés, qui sont décrits plus particulièrement dans le brevet US-A- 4 139 619, mais il reste, d'une manière générale, qu'il serait intéressant et utile de pouvoir disposer de composés actifs autres que ceux déja connus.

Or, la demanderesse vient maintenant de découvrir, après d'importantes recherches menées sur la question, que des composés de type α-Pyrone de formule décrite ci-après, permettent d'induire et de stimuler la croissance des cheveux, et/ou de diminuer leur chute, de manière efficace.

Cette découverte est à la base de la présente invention.

La présente invention a donc pour objet l'utilisation dans une composition cosmétique et/ou thérapeutique pour induire et stimuler la croissance des cheveux et/ou freiner leur chute dans un milieu physiologiquement acceptable, d'au moins une α-Pyrone répondant à la formule suivante: 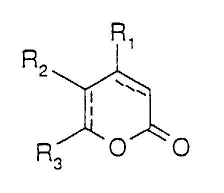 dans laquelle :
- R₁ représente un atome d'hydrogène ou un groupement alcoxy contenant de 1 à 4 atomes de carbone,
- R₂ représente un atome d'hydrogène ou un groupement hydroxyle,
- R₃ représente un groupement alkyle contenant de 1 à 4 atomes de carbone ou bien encore un groupement styryle ou phénétyle éventuellement substitués par un ou deux groupements méthylènedioxy ou un ou deux groupements hydroxyle et/ou groupements alcoxy contenant de 1 à 4 atomes de carbone,
étant entendu que lorsque R₂ designe un groupement hydroxyle, alors R₃ désigne obligatoirement un groupement phénétyle non substitué.

Ainsi, les α-Pyrones utilisés selon l'invention présentent soit des simples liaisons soit des doubles liaisons.

Un autre objet de l'invention concerne l'utilisation de cette composition pour la fabrication d'un médicament destiné au traitement par voie topique de la chute des cheveux.

D'autres caractéristiques et objets de l'invention apparaîtront à la lecture de la description et des exemples qui suivent.

Parmi les composés α-Pyrones utilisables dans le cadre de l'invention, on préfère mettre en oeuvre ceux pour lesquels :
- R₁ et R₂ désignent un atome d'hydrogène, et R₃ désigne un groupement méthyle, c'est à dire l'acide para-sorbique, encore appelé acide para-sorbinique, ou 5,6-dihydro 6-méthyl 2H-pyranne-2-one,
- R₁ et R₂ désignent un atome d'hydrogène, et R₃ désigne un groupement méthyle, c'est à dire la 5-méthyl δ-valérolactone, ou tétrahydro 6-méthyl 2H-pyranne-2-one,
- R₁ désigne un groupement méthoxy, R₂ désigne un atome d'hydrogène, et R₃ désigne un groupement styryle, c'est à dire la Kawaine ou 5,6-dihydro 4-méthoxy 6-(2-phényléthényl) 2H-pyranne-2-one,
- R₁ désigne un groupement méthoxy, R₂ désigne un atome d'hydrogène, et R₃ désigne un groupement styryle, c'est à dire la 5,6-Deshydrokawaine, ou 4-méthoxy 6-(2-phényléthényl) 2H-pyranne-2-one,
- R₁ désigne un groupement méthoxy, R₂ désigne un atome d'hydrogène, et R₃ désigne un groupement phénétyle, c'est à dire la 7,8-dihydrokawaine, ou Marindinine, ou 5,6-dihydro 4-méthoxy 6-(2-phényléthyl) 2H-pyranne-2-one,
- R₁ désigne un groupement méthoxy, R₂ désigne un groupement hydroxyle, et R₃ désigne un groupement phénétyle, c'est à dire le Dihydrokawaine-5-ol ou 5,6-dihydro 5-hydroxy 4-méthoxy 6-(2-phényléthyl) 2H-pyranne-2-one,
- R₁ désigne un groupement méthoxy, R₂ désigne un atome d'hydrogène, et R₃ désigne un groupement 3,4-méthylènedioxystyryle, c'est à dire la Méthysticine, ou 6-[2-(1,3-benzodioxol-5-yl) éthényl] 5,6-dihydro 4-méthoxy 2H-pyranne-2-one,
- R₁ désigne un groupement méthoxy, R₂ désigne un atome d'hydrogène, et R₃ désigne un groupement 3,4-méthylènedioxyphénétyle, c'est à dire la 7,8-dihydrométhysticine, ou 6-[2-(1,3-benzodioxol-5-yl) éthyl] 5,6 -dihydro 4-méthoxy 2H-pyranne-2-one,
- R₁ désigne un groupement méthoxy, R₂ désigne un atome d'hydrogène, et R₃ désigne un groupement 4-méthoxystyryle, c'est à dire la Yangonine, ou 4-méthoxy 6-[2-(4-méthoxyphényl) éthényl] 2H-pyranne-2-one,
- R₁ désigne un groupement méthoxy, R₂ désigne un atome d'hydrogène, et R₃ désigne un groupement 4-hydroxystyryle, c'est à dire la Noryangonine, ou 6-[2-(4-hyd roxyphényl) éthényl] 4-méthoxy 2H-pyranne-2-one,
- R₁ désigne un groupement méthoxy, R₂ désigne un atome d'hydrogène, et R₃ désigne un groupement 4-hydroxy 3-méthoxy styryle, c'est à dire la 11-Méthoxynoryangonine, ou 6-[2-(4-hydroxy 3-méthoxy phényl) éthényl] 4-méthoxy 2H-pyranne-2-one,
- R₁ désigne un groupement méthoxy, R₂ désigne un atome d'hydrogène, et R₃ désigne un groupement 3,4-diméthoxystyryle, c'est à dire la 11-Méthoxyyangonine, ou 6-[2-(3,4-diméthoxyphényl) éthényl] 4-méthoxy 2H-pyranne-2-one,
- R₁ désigne un groupement méthoxy, R₂ désigne un atome d'hydrogène, et R₃ désigne un groupement 4-méthoxyphénétyle, c'est à dire la 7,8-dihydroyangonine, ou 4-méthoxy 6-[2-(4-méthoxyphényl) éthyl] 2H-pyranne-2-one.

Dans la présente invention, la 5-méthyl δ-valérolactone et la Kawaine représentent les composés les plus particulièrement préférés.

Les composés de l'α-Pyrone de formule ci-dessus sont généralement utilisés, conformément à l'invention, dans des proportions comprises de préférence entre environ 0,05 et 10% en poids et plus particulièrement entre 0,1 et 6% en poids par rapport au poids total de la composition.

Tous ces composés sont bien connus dans la littérature.

La Kawaine et ses dérivés, la Méthysticine et ses dérivés, la Yangonine et ses dérivés, sont présents à l'état naturel dans la plante polynésienne Piper Methysticum ou Kawa-Kawa ; l'acide para-sorbique est notamment présent dans les baies du Sorbus Aucuparia et la 5-méthyl δ-valérolactone est notamment présente dans l'Avena Sativa. Ils peuvent en être extraits par les diverses méthodes bien connues d'extraction.

Ces composés peuvent également être synthétisés selon des procédés connus de la littérature.

Ainsi, pour la Kawaine et ses dérivés, on pourra se reporter aux références suivantes : FOWLER et al, J. CHEM. Soc., 1950, 3642 ; ISAWA T. et al, Chem. Lett., 1975, 161 ; ISRAILI ZH. et al, J. Org. Chem.,1976, 41, 4070 CASTELLINO S. et al, Tetrahedron Lett, 1984, 25, 4059 BELANGER A. et al, Can. J. Chem., 1975, 53, 201 ; SUSUKI E. et al, Synthesis, 1975, 192 ; ACHENBACH H. et al, Tetrahedron Lett, 1970, 3259, 1974, 119 ; HAENSEL R. et al, Chem. Ber., 1973, 106, 570.

Pour les autres composés, on pourra se reporter aux références suivantes:
- Méthysticine et ses dérivés : KLOHS M.W. et al, J. Org. Chem., 1959, 24, 1829 ;
- Yangonine et ses dérivés : HARRIS T.M. et al, J. Org. Chem., 1968, 33, 2399; HIPOLITO E. et al, Rev. Latinoam. Quim., 1977, 8, 79 ;
- Acide para-sorbique : HAYNES L.J. et al, J. Chem. Soc.,1946, 954 ; SATO T., Heterocycles, 1986, 24, 2173 ; STEVENSON R. et al, J. Nat. Prod. (Lloydia), 1988, 51, 1215 ; GOPALAN A.S. et al, Tetrahedron Lett., 31, 5575 ;
- 5-méthyl δ-valérolactone : PARLIMENT T.H. et al, Chem. Ind. (London), 1966, 1845 ; TAUB D. et al, Tetrahedron, 1968, 24, 2443 ; STURTZ G. et al, Tetrahedron Lett., 1976, 47 ; SEEBACH D. et al, Helv. Chim. Acta, 1985, 68, 2342 ; GIESE B. et al, J. Org. Chem.,1986, 51, 3726.

Le milieu physiologiquement acceptable attaché aux compositions selon l'invention peut être anhydre ou au contraire aqueux. On entend par milieu anhydre, un milieu solvant contenant moins de 1% d'eau. Ce milieu peut être constitué d'un solvant ou d'un mélange de solvants choisi plus particulièrement parmi les alcools inférieurs en C₂-C₄ comme l'alcool éthylique, les alkylèneglycols comme le propylèneglycol, et les alkyléthers d'alkylèneglycols ou de dialkylèneglycols, dont les radicaux alkyle ou alkylène contiennent de 1 à 4 atomes de carbone. On entend par milieu aqueux, un milieu constitué par de l'eau ou un mélange d'eau et d'un autre solvant physiologiquement acceptable, choisi notamment parmi les solvants organiques cités ci-dessus. Dans ce dernier cas, ces autres solvants, lorsqu'ils sont présents, représentent environ 5 à 95% en poids de la composition.

La composition peut contenir d'autres adjuvants habituellement utilisés dans le domaine cosmétique ou pharmaceutique, en vue de réaliser des compositions topiques, tels que des agents tensio-actifs, des agents épaississants ou gélifiants, des agents cosmétiques, des agents conservateurs, des agents alcalinisants ou acidifiants bien connus dans l'état de la technique, et en quantités suffisantes pour obtenir la forme de présentation désirée, notamment de lotion plus ou moins épaissie, de gel, d'émulsion, ou de crême. La composition peut éventuellement aussi être pressurisée en aérosol ou vaporisée à partir d'un flacon pompe.

Lorsque la composition est épaissie ou gélifiée à l'aide d'un agent épaississant, ce dernier est généralement présent dans des concentrations comprises entre environ 0,1 et 6% par rapport au poids total de la composition.

Elle peut être utilisée notamment dans les traitements mettant en oeuvre une application qui est suivie ou non suivie d'un rinçage, ou encore sous forme de shampooing.

Le pH de la composition peut varier entre environ 3 et 9 et de préférence entre 5 et 8.

La composition selon l'invention peut contenir, en plus de l'α-Pyrone, des composés améliorant encore l'activité sur la repousse et/ou sur le freinage de la chute des cheveux, et ayant déjà été décrits pour cette activité.

Parmi ces derniers composés, on peut plus particulièrement citer à titre non limitatif :
- les esters d'acide nicotinique, dont notamment le nicotinate de tocophérol, le nicotinate de benzyle et les nicotinates d'alkyles en C₁-C₆ comme les nicotinates de méthyle ou d'hexyle ;
- les agents anti-inflammatoires stéroidiens et non stéroidiens, en particulier, l'hydrocortisone, ses sels et ses dérivés, l'acide niflumique, et autres ;
- les rétinoides, comme l'acide t-trans rétinoique, appelé encore Trétinoine, l'Isotrétinoine, le Rétinol ou vitamine A, et ses dérivés, tels que l'acétate, le palmitate ou le propionate, le Motrétinide, l'Etrétinate, le t-trans rétinoate de zinc ;
- les dérivés de pyrimidine, comme le 6-amino 1,2-dihydro 1-hydroxy 2-imino 4-pipéridino pyrimidine encore connu sous le nom de Minoxidil, et tels que décrits dans le brevet US-A- 4 139 619 ;
- les agents antibactériens tels que les macrolides, les pyranosides et les tétracyclines, et notamment l'Erythromycine ;
- les agents antagonistes de calcium, comme la Cinnarizine et le Diltiazem ;
- des hormones, telles que l'estriol ou des analogues, ou la thyroxine et ses sels ;
- des agents antiandrogènes, tels que l'oxendolone, la spironolactone, le diéthylstilbestrol ;
- des inhibiteurs des 5-α-réductases ;
- des capteurs de radicaux OH, tels que le diméthylsulfoxyde ;

A la liste ci-dessus, d'autres composés peuvent également être rajoutés, à savoir par exemple le Diazoxyde, la Spiroxazone, des phospholipides comme la lécithine,les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position-5 du cycle benzénique, des acides hydroxycarboxyliques ou cétocarboxyliques et leurs esters, des lactones et leurs sels correspondants, I'Anthraline, des caroténoides, les acides eicosatétraynoïque et eicosatriynoïque ou leurs esters et amides.

Le procédé de traitement de la chute des cheveux consiste à appliquer sur les zones alopéciques du cuir chevelu et des cheveux d'un individu, une quantité efficace d'une composition telle que définie ci-dessus, à laisser en contact plusieurs heures et éventuellement à rincer. On peut, par exemple, appliquer la composition sur les cheveux et le cuir chevelu, le soir, garder la composition au contact toute la nuit et éventuellement effectuer un shampooing le matin, ou laver les cheveux à l'aide de cette composition et laisser à nouveau au contact quelques minutes avant de rincer. Ces applications peuvent être renouvellées quotidiennement pendant un ou plusieurs mois suivant les individus.

Le procédé de traitement présente les caractéristiques d'un procédé cosmétique dans la mesure où il permet d'améliorer l'esthétique des cheveux en leur donnant une plus grande vigueur et un aspect meilleur.

L'invention a également pour objet l'utilisation des α-Pyrones de formule définie ci-dessus pour la préparation d'un médicament destiné au traitement des maladies du cuir chevelu telles que l'alopécie.

Les exemples qui suivent illustrent l'invention.

### Exemple 1

On prépare une lotion antichute de composition suivante :
- Kawaine 1,0.g
- Propylène glycol 10,0 g
- Alcool isopropylique qsp 100,0 g

On applique 1 ml de cette lotion sur le cuir chevelu, à la fréquence de une à deux fois par jour.

### Exemple 2

On prépare une lotion antichute de composition suivante :
- 5-méthyl δ-valerolactone 1,5 g
- Propylène glycol 30,0 g
- Alcool éthylique 40,5 g
- Eau qsp 100,0 g

On applique cette lotion sur le cuir chevelu, une à deux fois par jour, à raison d' 1ml par application.

### Exemple 3:

On prépare une lotion épaissie antichute de composition suivante :
- 5-méthyl δ-valerolactone 1,0 g
- Kawaine 2,0 g
- Hydroxypropylcellulose vendue par la société Hercules sous la dénomination Klucel G 3,5 g
- Alcool éthylique qsp 100,0 g

On applique cette lotion épaissie sur le cuir chevelu, une à deux fois par jour, à raison d' 1ml par application.

### Exemple 4

On prépare une lotion épaissie antichute de composition suivante :
- Méthysticine 0,5 g
- Monométhyléther de propylèneglycol vendu sous la dénomination Dowanol PM par la société Dow Chemical 20,0 g
- Hydroxypropylcellulose vendue par la société Herculès sous la dénomination Klucel G 3,0 g
- Alcool éthylique 40,0 g
- Eau qsp 100,0 g

On applique cette lotion épaissie sur le cuir chevelu, une à deux fois par jour, à raison d' 1ml par application.

Avec chacune des compositions décrites dans les exemples 1 à 4 ci-dessus, on a constaté, après plusieurs mois de traitement et selon les sujets traités, un ralentissement de la chute des cheveux et/ou un effet repousse.

## Revendications

1. Utilisation d'au moins un composé α-Pyrone répondant à la formule suivante : 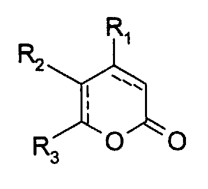 dans laquelle :
- R₁ représente un atome d'hydrogène ou un groupement alcoxy contenant de 1 à 4 atomes de carbone,
- R₂ représente un atome d'hydrogène ou un groupement hydroxyle,
- R₃ représente un groupement alkyle contenant de 1 à 4 atomes de carbone ou bien encore un groupement styryle ou phénétyle éventuellement substitués par un groupement méthylènedioxy ou un groupement hydroxyle et/ou un ou deux groupements alcoxy contenant de 1 à 4 atomes de carbone,
étant entendu que lorsque R₂ désigne un groupement hydroxyle, alors R₃ désigne obligatoirement un groupement phénétyle non substitué,
dans une composition cosmétique à usage topique ou pour la préparation d'une composition pharmaceutique à usage topique, dans un milieu physiologiquement acceptable, destinée à induire et stimuler la croissance des cheveux et/ou freiner leur chute

2. Utilisation selon la revendication 1, caractérisée par le fait que le composé α-Pyrone est choisi parmi la Kawaine et la 5-méthyl δ-valérolactone.

3. Utilisation selon les revendications 1 ou 2, caractérisée par le fait que le milieu physiologiquement acceptable est un milieu approprié en cosmétique et/ou en pharmacie, et qu'il est constitué par de l'eau, ou un solvant organique, ou un mélange d'eau et d'un solvant organique, et qu'il est épaissi ou gélifié, ou non.

4. Utilisation selon la revendication 3, caractérisée par le fait que le solvant, dans le cas du mélange, est présent dans des proportions comprises entre environ 5 et 95% en poids par rapport au poids total de la composition.

5. Utilisation selon la revendication 3, caractérisée par le fait que la composition comprend un agent épaississant dans des concentrations comprises entre environ 0,1 et 6% par rapport au poids total de la composition.

6. Utilisation selon l'une quelconque des revendications précédentes, caractérisée par le fait qu'elle comprend en outre d'autres agents connus comme ayant une activité sur la repousse des cheveux et/ou le freinage de leur chute.

## Claims

1. Use of at least one α-pyrone compound corresponding to the following formula: 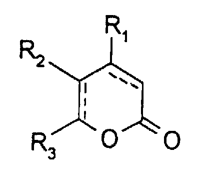 in which:
- R₁ represents a hydrogen atom or an alkoxy group containing from 1 to 4 carbon atoms,
- R₂ represents a hydrogen atom or a hydroxyl group,
- R₃ represents an alkyl group containing from 1 to 4 carbon atoms or alternatively a styryl or phenethyl group optionally substituted with a methylenedioxy group or a hydroxyl group and/or one or two alkoxy groups containing from 1 to 4 carbon atoms,
on the understanding that, when R₂ denotes a hydroxyl group, then R₃ necessarily denotes an unsubstituted phenethyl group,
in a cosmetic composition for topical use or for the preparation of a pharmaceutical composition for topical use, in a physiologically acceptable medium, intended to induce and stimulate hair growth and/or retard hair loss.

2. Use according to Claim 1, characterized in that the α-pyrone compound is chosen from kavain and 5-methyl-δ-valerolactone.

3. Use according to Claim 1 or 2, characterized in that the physiologically acceptable medium is a medium suitable for use in cosmetics and/or in pharmacy, and in that it consists of water or an organic solvent or a mixture of water and an organic solvent, and in that it is thickened or gelled or otherwise.

4. Use according to Claim 3, characterized in that the solvent, in the case of the mixture, is present in proportions of between approximately 5 and 95% by weight relative to the total weight of the composition.

5. Use according to Claim 3, characterized in that the composition comprises a thickening agent in concentrations of between approximately 0.1 and 6 % relative to the total weight of the composition.

6. Use according to any one of the preceding claims, characterized in that it comprises, in addition, other agents known to have activity with respect to hair regrowth and/or the retarding of hair loss.

## Patentansprüche

1. Verwendung mindestens einer α-Pyron-Verbindung der folgenden Formel 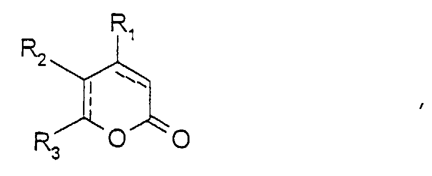 in der bedeuten:
- R₁ Wasserstoff oder eine Alkoxygruppe mit 1 bis 4 Kohlenstoffatomen,
- R₂ Wasserstoff oder eine Hydroxygruppe,
- R₃ eine Alkylgruppe mit 1 bis 4 Kohlenstoffatomen oder eine Styrylgruppe oder eine Phenetylgruppe, die gegebenenfalls mit einer oder zwei Methylendioxygruppen oder einer Hydroxygruppe und/oder einer oder zwei Alkoxygruppen mit 1 bis 4 Kohlenstoffatomen substituiert sind,
mit der Maßgabe, daß R₃ obligatorisch eine nicht substituierte Phenetylgruppe bedeutet, wenn R₂ eine Hydroxygruppe ist,
in einer kosmetischen Zusammensetzung zur topischen Anwendung oder für die Herstellung einer pharmazeutischen Zusammensetzung zur topischen Anwendung in einem physiologisch akzeptablen Medium, die dafür vorgesehen ist, das Haarwachstum zu induzieren oder zu stimulieren und/oder den Haarausfall zu verlangsamen.

2. Verwendung nach Anspruch 1, dadurch gekennzeichnet, daß die α-Pyron-Verbindung unter Kavain und 5-Methyl-δ-valerolacton ausgewählt wird.

3. Verwendung nach Anspruch 1 oder 2, dadurch gekennzeichnet, daß das physiologisch akzeptable Medium ein kosmetisch und/oder pharmazeutisch geeignetes Medium ist, daß es aus Wasser, einem organischen Lösungsmittel oder einem Gemisch aus Wasser und einem organischen Lösungsmittel besteht und daß es gegebenenfalls verdickt oder in Form eines Gels vorliegt.

4. Verwendung nach Anspruch 3, dadurch gekennzeichnet, daß das Lösungsmittel im Falle des Gemischs in einem Anteil von etwa 5 bis 95 Gew.-%, bezogen auf das Gesamtgewicht der Zusammensetzung, enthalten ist.

5. Verwendung nach Anspruch 3, dadurch gekennzeichnet, daß die Zusammensetzung ein Verdickungsmittel in einer Konzentration von etwa 0,1 bis 6 Gew.-%, bezogen auf das Gesamtgewicht der Zusammensetzung, enthält.

6. Verwendung nach einem der vorhergehenden Ansprüche, dadurch gekennzeichnet, daß die Zusammensetzung außerdem andere Mittel enthält, die dafür bekannt sind, daß sie eine den Haarwuchs fördernde und/oder den Haarausfall verlangsamende Wirkung haben.
